# EUROPEAN PATENT SPECIFICATION

(11) **EP 4 393 913 B1**
(45) Date of publication and mention of the grant of the patent: **29.10.2025**
(21) Application number: 23220433.9
(22) Date of filing: 28.12.2023
(51) Int. Cl.: C07D 311/04

(54) **PROCESS FOR THE PREPARATION OF DELTA-TOCOTRIENOL**
VERFAHREN ZUR HERSTELLUNG VON DELTA-TOCOTRIENOL
PROCÉDÉ DE PRÉPARATION DE DELTA-TOCOTRIÉNOL

(30) Priority: 30.12.2022 WO PCT/US2022/082646
(43) Date of publication of application: 03.07.2024
(73) Proprietor: Peptinovo Biopharma Inc., Ann Arbor, MI 48103 (US)
(72) Inventor: CASTALDI, Graziano, 28072 ITALY (IT); CASTALDI, Michele, 21029 ITALY (IT); ZAMBON, Michele, 28072 ITALY (IT)
(74) Representative: Ungria López, Javier

(56) References cited:
- WO-A1-2019/053605
- WO-A2-2005/035490
- CHENEVERT R ET AL: "Chemoenzymatic synthesis of both enantiomers of @a-tocotrienol", BIOORGANIC & MEDICINAL CHEMISTRY, ELSEVIER, AMSTERDAM, NL, vol. 14, no. 15, 1 August 2006 (2006-08-01), pages 5389 - 5396, XP027992806, ISSN: 0968-0896, [retrieved on 20060801]
- ELIAS A. COULADOUROS ET AL: "A Short and Convenient Chemical Route to Optically Pure 2-Methyl Chromanmethanols. Total Asymmetric Synthesis of ?-, ?-, and ?-Tocotrienols", THE JOURNAL OF ORGANIC CHEMISTRY, vol. 72, no. 18, 1 August 2007 (2007-08-01), pages 6735 - 6741, XP055469387, ISSN: 0022-3263, DOI: 10.1021/jo0705418

## Description

### BACKGROUND OF THE INVENTION

Vitamin E, also known as Tocopherol, is a fat-soluble vitamin introduced into the organism through food. It is accumulated in the liver and released in small doses from the body when its use become necessary.

It is abundant in foods, especially in oil fruits, like olives, peanuts and corn, and in wheat seeds. It can be found also in cereals, nuts and green vegetables.

Vitamin E is the major lipid-soluble component in the cell antioxidant defence system. It has the ability to protect cells from free radical damage as well as to reduce the production of free radicals in certain situations. These features make it an important cancer prevention tool. In addition, Vitamin E has been found to be very effective in the prevention and reversal of various disease complications due to its function as an antioxidant, its role in antiinflammatory processes, its inhibition of platelet aggregation and its immune-enhancing activity.

This vitamin occurs naturally in eight main isoforms: α, β, γ, and δ-tocotrienols, and four corresponding tocopherols. Tocotrienols differ from tocopherols by bearing a farnesyl (three double bonds) moiety rather than a saturated phytyl side chain. α, β, γ, and δ-homologues are defined by the methylation patterns of the aromatic ring.

Tocotrienols have been shown to have positive effects on various aspects of human health: down-regulate cholesterol biosynthesis by increasing HMG-CoA reductase degradation as well as decreasing the efficiency of the translation of HMG-CoA reductase mRNA; they are lipid-soluble antioxidants that protect membranes and cell components from the oxidative stress mediated by free radicals and also inhibit the oxidation of low-density lipoproteins associated with cardiovascular diseases. In addition, tocotrienols have important neuroprotective and antitumor properties: among the isoforms, δ-Tocotrienols is probably the most studied in the context of oncological pathologies. In fact, *in vitro* and preclinical studies have shown that δ-tocotrienol is the most active in preventing the proliferation of human melanoma cells.

Tocotrienols are plant constituents particularly abundant in palm oil and cereal seeds. Other cultivated plants that are rich in Tocotrienols includes rice, wheat, barley, rye and oat. Tocotrienols are present, in different levels, in various other oils, seed and fruits that are naturally occurring.

Considering the importance of tocotrienols in the management of health and due to varying levels of their occurrence in various natural sources and foods; these molecules often have to be supplemented.

However, there are some problems in the isolation of δ-tocotrienol from natural sources. Firstly, there is a limited and inadequate supply of raw materials, such as vegetable oils or seeds. Secondly, isolation from natural resources would require several preparative scale reverse-phase chromatography or expensive methodologies that involve critical distillation procedures or simulated moving bed chromatography. These limitations led to the development of various synthetic routes that gave access to the δ-tocotrienol and its derivatives.

δ-tocotrienol is a compound of formula (I): 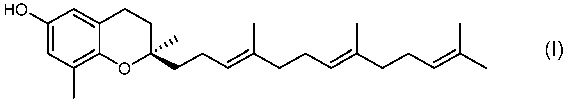 chemically known as (2R)-2,8-dimethyl-2-[(3E,7E)-4,8,12-trimethyltrideca-3,7,11-trienyl]-3,4-dihydrochromen-6-ol.

It has an absolute (2R) configuration on the chiral carbon of the chroman ring and three double bond sites at the 3', 7' and 11' positions of the 16-carbon chain attached to the chroman ring.

Various δ-tocotrienol processes are known in the literature which mostly share a similar synthetic approach.

In particular, δ-tocotrienol can be obtained by a coupling reaction between 8-methylcroman-2-methanol (MCM) of formula (II) 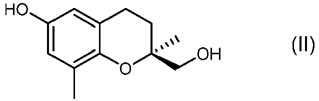 and the 15-carbon farnesyl chain of formula (III) 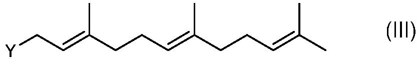 wherein Y represents a leaving group.

This reaction carried out in the presence of n-butyllithium and hexamethylphosphoramide, provides for the formation of a carbanion in alpha position with respect to the leaving group Y and its subsequent attack to the chroman derivative of formula (II).

Following the same synthetic approach, R. Chenevert et al. in Bioorg. Med. Chem. 2006, 14, 5389-5396 discloses a process for the preparation of tocotrienol in the α-isoform, as reported in the scheme 1 below:

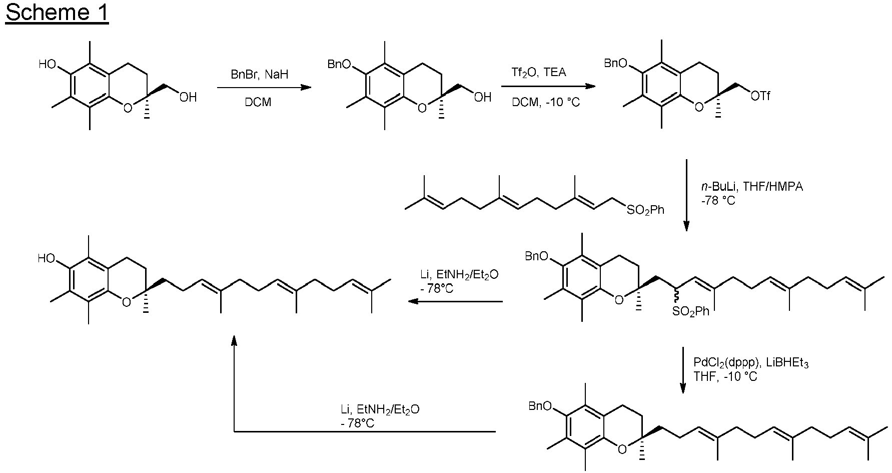

E. Couladouros et al. in J. Org. Chem. 2007, 72, 6735-6741 discloses a method for preparing β-, γ- e δ-tocotrienols according to the route of synthesis reported in Scheme 2 below:

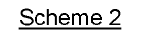

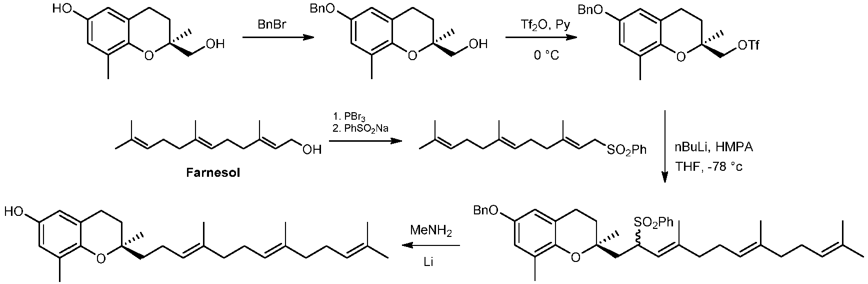

Similarly, the international patent application WO 2019/053605 discloses the process reported in Scheme 3:

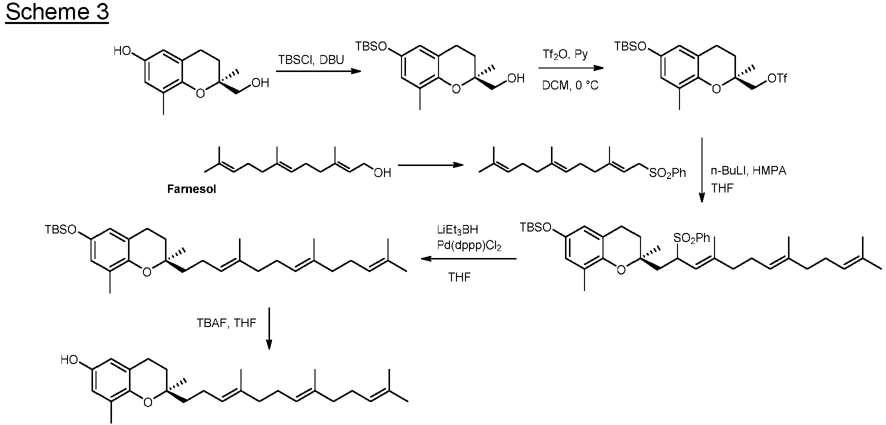

In addition, the international patent application WO 2005/035490 discloses a process for the synthesis of δ-tocotrienol in which the protected chroman sulfonate is reacted with a Grignard reagent prepared from a farnesyl halide according to the synthetic pathway reported below in Scheme 4: 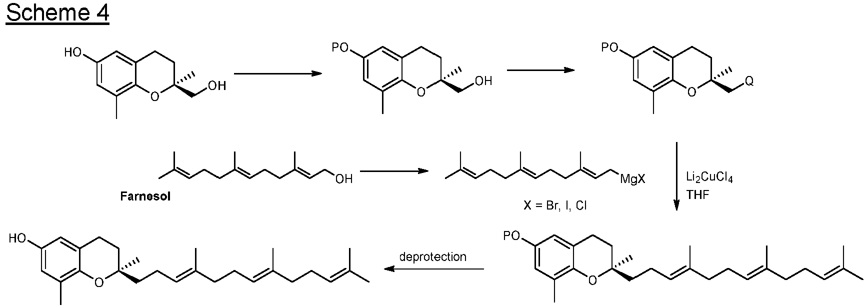 wherein P is a protecting group and Q is a sulfonate derivative.

However, the processes disclosed in the prior art are difficult to implement at an industrial scale since they require critical reaction conditions such as very low temperatures (-78°C), hazardous reagents as for example metallic lithium and toxic substances such as hexamethylphosphoramide, whose use is not allowed on large scale.

We have now found a new synthetic approach for the preparation of δ-tocotrienol, consisting in reacting directly the chroman derivative with the farnesyl derivative without making use of toxic reagents or low temperatures. This process of synthesis allows to obtain the desired product with high yields and purities, as well as reduced costs due to a more simplified chemistry.

Therefore, an object of the present invention is a process for the preparation of δ-tocotrienol of formula (I), 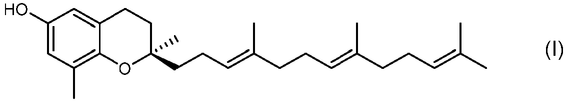 comprising:
c) reacting a compound of formula (V) 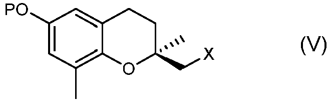
with a compound of formula (VII) 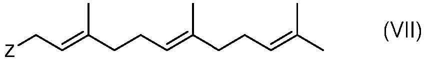
wherein P represents a protecting group selected from benzoyl, acetyl, trimethylsilyl, tert-butyldimethylsilyl, tert-butyldiphenylsilyl, p-toluenesulfonate, methansulfonate, benzensulfonate, p-toluenesulfonate ester, benzensulfonate ester, methanesulfonate ester, benzyl ether, methyl ether, 2-tetrahydropyranyl ether, 2-tetrahydrofuranyl ether, methoxymethyl ether,
X is a halogen atom selected from bromine, iodine, chlorine,
Z represents a halogen atom selected from bromine, iodine, chlorine
in the presence of magnesium metal, a magnesium-activating agent, and an optional additive,
to provide the compound of formula (VIII) 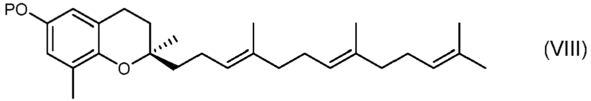 and
d) deprotecting the obtained compound of formula (VIII) to give the desired δ-tocotrienol of formula (I).

According to the process of the present invention, the coupling reaction of step c) between the chroman derivative of formula (V) and the farnesyl derivative of formula (VII) is carried out in an inert organic solvent in the presence of magnesium metal, a magnesium-activating agent, and an optional additive.

Examples of inert organic solvents according to the invention are diethyl ether, tetrahydrofuran, 2-methyl tetrahydrofuran, toluene or mixtures thereof.

Preferably the coupling reaction is performed in 2-methyl tetrahydrofuran.

In the process of the present invention, magnesium metal is introduced into the reaction mixture containing the chroman derivative of formula (V) and the farnesyl derivative of formula (VII) dissolved in the inert organic solvent.

Preferably, magnesium metal is added in an amount comprised between 1.0 and 10.0 molar equivalents, more preferably between 5.0 and 7.0 molar equivalents, with respect to the molar amount of the compound of formula (V).

In fact, the Applicant of the present invention has surprisingly found that the Grignard reagent formed by the reaction between magnesium metal and the chroman derivative of formula (V) could not be obtained under well-known experimental conditions (see comparative example) and that the coupling reaction only occurred when both single compounds were reacted in the presence of magnesium metal.

In addition, contrary to the experimental conditions disclosed in the prior art, in particular in WO 2005/035490, the coupling reaction is performed at higher temperatures, in particular at a temperature comprised between 20°C and 100°C, preferably of about 80°C.

The optional additive is a lithium, zinc, cobalt, nickel, copper, palladium, or iron salt selected from lithium chloride, lithium bromide, lithium iodide, zinc chloride, zinc bromide, zinc iodide, cobalt chloride, cobalt dichloride, nickel chloride, copper(I) chloride, copper(II) chloride, copper(I) bromide, copper(II) dibromide, copper iodide, copper triflate, palladium chloride, iron(III) chloride, preferably lithium chloride, and/or an organic chelating compound selected from tetramethylethylenediamine (TMEDA), 1,2-Dimethylethylenediamine (DMEDA), ethanolamine (ETA), triphenylphosphine (PPh₃), 1,3-bis(diphenylphosphino)propane (DPPP), 1,3-butadiene, isoprene, preferably TMEDA.

Said optional additive is preferably used in an amount comprised between 0.1 and 3.0 molar equivalents, with respect to the molar amount of the compound of formula (V).

The magnesium activating agent is selected from the group consisting of iodine, dichloroethane, dibromoethane, trimethylsilyl chloride, and is added to the reaction mixture in an amount comprised between 0.01 and 1.0 molar equivalents, with respect to the molar amount of the compound of formula (V).

Once the coupling reaction is complete, the compound of formula (VIII) is obtained and separated from the reaction mixture, according to techniques well known to the skilled person, such as extraction, filtration, crystallization, precipitation.

This compound is subsequently subjected to deprotection which can be performed in different conditions, depending on the protecting group P present on the aromatic moiety to afford the desired δ-tocotrienol of formula (I).

As it is well known to the skilled person, the deprotection is carried out in presence of an acid or a base selected from sodium hydroxide, potassium hydroxide, hydrochloric acid, sulfuric acid, acetic acid, formic acid in a solvent selected from a group consisting of water, alcohols, ethers, ketones or mixture of them, at a temperature comprised between 25°C and 80°C.

According to an embodiment of the present invention, the compound of formula (VII) 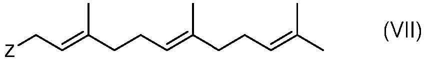 wherein Z represents a halogen atom selected from chlorine, bromine, iodine, preferably bromine is obtained by halogenating the compound of formula (IX), which is commercially available.

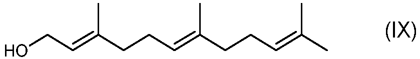

In the presence of a halogenating agent.

Typically, examples of halogenating agents that can be used in the process of the present invention are phosphorus tribromide, phosphorus oxychloride, phosphorus trichloride, phosphorus pentachloride, thionyl chloride in a aprotic solvent selected from N,N-dimethylformamide, tetrahydrofuran, dichloromethane.

In alternative, iodine and bromine can be used as halogenating agents in the presence of triphenylphosphine and imidazole.

According to a further embodiment, the present invention also relates to a process for the synthesis of the chroman derivative of formula (V) 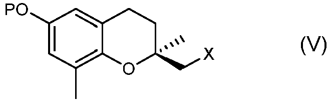 which comprises the following steps:
a) Selectively protecting the aromatic hydroxyl group of 8-methylcroman-2-methanol of formula (II) in single enantiomer form 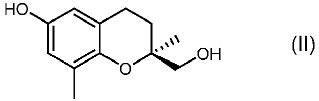 in the presence of a protecting agent, to give the intermediate of formula (IV) 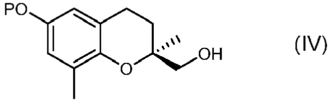 wherein P has the above reported meanings; and
b) converting the compound of formula (IV) into the corresponding halide derivative of formula (V) 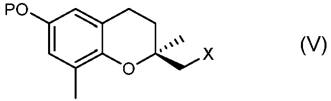 wherein X is a halogen atom selected from bromine, iodine, chlorine, preferably iodine.

According to the present invention, step a) is carried out by dissolving the compound of formula (II), i.e. 8-methylcroman-2-methanol in an aprotic solvent.

Examples of aprotic solvents that can be used in this reaction are N,N-dimethylformamide, tetrahydrofuran, dichloromethane.

Preferably, 8-methylcroman-2-methanol is dissolved in dichloromethane.

A protecting agent of formula P-X, wherein P has the above-mentioned meanings and X is a halogen atom, is introduced in the reaction mixture in the presence of a base.

Preferred bases according to the present invention are selected from triethylamine, tributylamine, N,N-diisopropylethylamine, 4-dimethylaminopyridine, potassium carbonate, sodium carbonate, more preferably triethylamine.

Examples of protecting agents that can be used so as to introduce the P group having the above-mentioned meanings are tosyl chloride and benzyl bromide.

Preferably the protecting agent is used in an amount comprised between 1.0 and 2.0 molar equivalents, more preferably of about 1.1 molar equivalents, with respect to the molar amount of the compound of formula (II).

The compound of formula (IV) thus obtained is subsequently halogenated under experimental conditions well known to the skilled person in order to obtain the desired chroman derivative of formula (V).

In alternative, the compound of formula (V) can be prepared according to a process comprising the following steps:
b1) converting the compound of formula (IV)

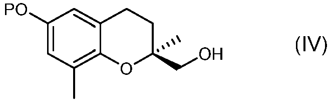

Into the corresponding compound of formula (VI)

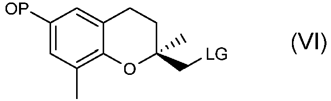

Wherein P has the above-mentioned meanings
And LG represents a sulfonate leaving group selected from tosyl, mesyl, nosyl, triflate, nonaflate, preferably tosyl;
b2) halogenating the compound of formula (VI) thus obtained to provide the desired compound of formula (V).

Preferably, the chroman derivative of formula (V) is prepared by first converting the compound of formula (IV) to an intermediate compound of formula (VI), and then converting the compound of formula (VI) into the corresponding halide.

According to a most preferred embodiment, the present invention is directed to a process for the preparation of δ-tocotrienol of formula (I) 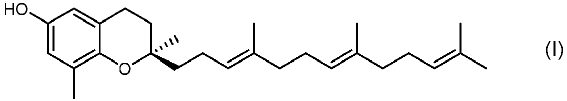 comprising the following steps:
a) selectively protecting the aromatic hydroxyl group of 8-methylcroman-2-methanol of formula (II) in single enantiomer form 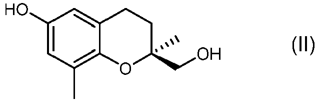 in the presence of a protecting agent, to give the intermediate of formula (IV) 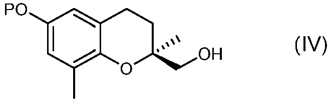 wherein P represents a protecting group selected from benzoyl, acetyl, trimethylsilyl, tert-butyldimethylsilyl, tert-butyldiphenylsilyl, p-toluenesulfonate, methansulfonate, benzensulfonate, p-toluenesulfonate ester, benzensulfonate ester, methanesulfonate ester, benzyl ether, methyl ether, 2-tetrahydropyranyl ether, 2-tetrahydrofuranyl ether, methoxymethyl ether,
b1) converting the compound of formula (IV) into the corresponding compound of formula (VI) 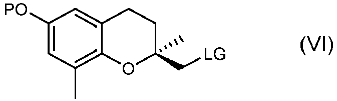 wherein P and LG have the above-reported meanings,
b2) halogenating the compound of formula (VI) thus obtained to provide the compound of formula (V) 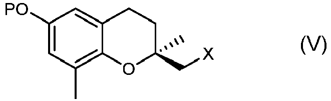 wherein P has the above-reported meanings, X is a halogen atom selected from bromine, iodine, chlorine,
c) reacting the compound of formula (V) with a compound of formula (VII) 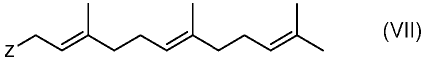
   Z represents a halogen atom selected from bromine, iodine, chlorine in the presence of magnesium metal, a magnesium-activating agent and an optional additive,
   to provide the compound of formula (VIII) 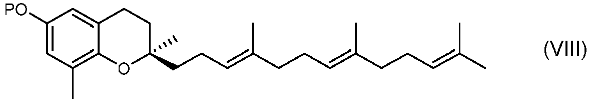 and
d) deprotecting the obtained compound of formula (VIII) to give the desired δ-tocotrienol of formula (I).

The process object of the present invention can be schematized as reported in Scheme 5 below.

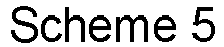

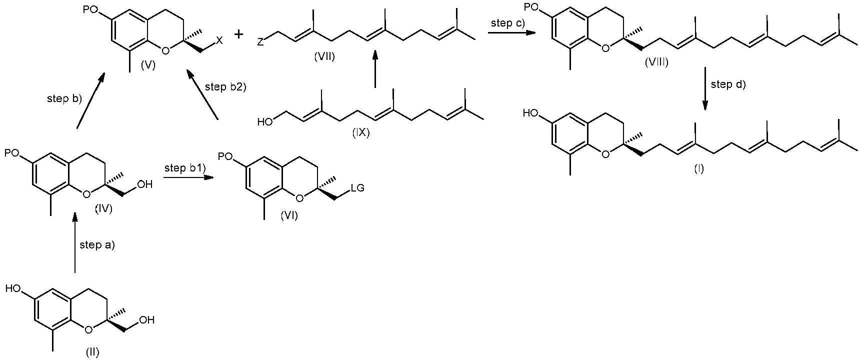

The present invention will now be illustrated by means of some examples, which should not be seen as limiting the scope of the invention.

### EXAMPLES

### EXAMPLE 1. Synthesis of (S)-(6-(benzyloxy)-2,8-dimethylchroman-2-yl)methanol.

In a reaction flask 20.0 g of 8-methylcroman-2-methanol (MCM, 1 eq), 100 mL of dimethylformamide (5 vol), 19.9 g of potassium carbonate (1.5 eq) and 18.1 g of benzyl bromide (1.1 eq) are charged.

The reaction mixture is left under stirring at room temperature overnight.

At the end of the reaction, 150 mL of water and 150 mL of ethyl acetate are added and the organic phase is washed with water (3 x 100 mL) and brine (1x 50 mL). The collected organic phase is filtered on celite and charcoal and then dried with magnesium sulphate. After evaporation of the solvent, 28.42 g of (S)-(6-(benzyloxy)-2,8-dimethylchroman-2-yl)methanol is obtained (99.3%).

¹H-NMR (CDCl₃, 400 MHz): δ 7.46-7.34 (m, 5H), δ 6.70-6.69 (d, 1H), δ 6.58-6.57 (d, 1H), δ 5.00 (s, 2H), δ 3.69-3.59 (m, 2H) δ 2.89-2.71 (m, 2H), δ 2.18 (s, 3H), δ 2.07-1.96 (m, 2H), δ 1.74-1.65 (m, 1H), δ 1.27 (s, 1H).

¹³C-NMR (CDCl₃, 400 MHz): δ 151.87, δ 145.73, δ 137.59, δ 128.54, δ 127.82, δ 127.49, δ 127.19, δ 121.11, δ 115.97, δ 112.32, δ 70.58, δ 69.41, δ 27.81, δ 22.29, δ 20.84, δ 16.28.

### EXAMPLE 2. Synthesis of (S)-(6-(benzyloxy)-2,8-dimethylchroman-2-yl)methyl 4-methylbenzenesulfonate.

In a reaction flask 25.0 g of (S)-(6-(benzyloxy)-2,8-dimethylchroman-2-yl)methanol (1 eq), 250 mL of dichloromethane (10 vol), 18.8 g of 4-dimethylaminopyridine (DMAP, 2 eq) are charged.

The reaction mixture is cooled to 10 °C and 19.1 g of tosyl chloride (1.2 eq) are added. The reaction is left under stirring at room temperature overnight.

At the end of the reaction, 50 mL of water are added. The organic phase is washed with a 6 N solution of hydrochloric acid (1 x 20 mL) and water (2x 50 mL) to reach a pH value of 6. The collected organic phase is dried with magnesium sulphate. After evaporation of the solvent, 37.63 g of (S)-(6-(benzyloxy)-2,8-dimethylchroman-2-yl)methyl 4-methylbenzenesulfonate is obtained (99.2%).

¹H-NMR (CDCl₃, 400 MHz): δ 7.84-7.82 (d, 2H), δ 7.45-7.35 (m, 7H), δ 6.69-6.68 (d, 1H), δ 6.54-6.53 (d, 1H), δ 5.00 (s, 2H), δ 4.07-3.95 (dd, 2H), δ 2.72-2.69 (m, 2H), δ 2.48 (s, 3H), δ 2.11 (s, 3H), δ 1.95-1.93 (m, 1H), δ 1.80-1.76 (m, 1H), δ 1.34 (s, 3H).

¹³C-NMR (CDCl₃, 400 MHz): δ 152.00, δ 145.25, δ 144.95, δ 137.53, δ 132.75, δ 129.94, δ 128.56, δ 127.95, δ 127.85, δ 127.48, 127.31, δ 120.55, δ 116.10, δ 112.13, δ 73.97, δ 73.51, δ 70.52, δ 28.16, δ 22.38, δ 21.93, δ 21.68, δ 16.14.

### EXAMPLE 3. Synthesis of (S)-6-(benzyloxy)-2-(iodomethyl)-2,8-dimethylchromane.

In a reaction flask 2.9 g of (S)-(6-(benzyloxy)-2,8-dimethylchroman-2-yl)methyl 4-methylbenzenesulfonate (1 eq), 29 mL of dimethylformamide (10 vol) and 5.3 g of potassium iodide (5 eq) are charged. The temperature is brought at 150 °C and the reaction mixture is left under stirring in these conditions for about 10 hours.

At the end of the reaction, 30 mL of water and 30 mL of ethyl acetate are added, the phases are separated and the aqueous phase is extracted with 20 mL of ethyl acetate. Then, the organic phases are combined and washed with water (3 x 50 mL) and brine (1 x 20 mL).

The collected organic phase is filtered on celite and charcoal and then dried with magnesium sulphate. After evaporation of the solvent, 2.34 g of (S)-6-(benzyloxy)-2-(iodomethyl)-2,8-dimethylchromane is obtained (89.6%).

¹H-NMR (CDCl₃, 400 MHz): δ 7.46-7.34 (m, 5H), δ 6.71-6.70 (d, 1H), δ 6.58-6.57 (d, 1H), δ 5.01 (s, 2H), δ 3.36 (m, 2H), δ 2.76-2.73 (m, 2H), δ 2.19 (s, 3H), δ 2.13-2.08 (m, 1H), δ 1.96-1.91 (m, 1H), δ 1.49 (s, 3H).

¹³C-NMR (CDCl₃, 400 MHz): δ 152.08, δ 145.62, δ 137.63, δ 128.59, δ 127.87, δ 127.63, δ 127.53, δ 120.61, δ 116.16, δ 112.20, δ 73.71, δ 70.58, δ 30.28, δ 25.60, δ 22.61, δ 16.39, δ 15.24.

### EXAMPLE 4. Synthesis of (S)-6-(benzyloxy)-2-(bromomethyl)-2,8-dimethylchromane.

In a reaction flask 4.2 g of (S)-(6-(benzyloxy)-2,8-dimethylchroman-2-yl)methyl 4-methylbenzenesulfonate (1 eq), 40 mL of dimethylformamide (10 vol) and 12.0 g of sodium bromide (15 eq) are charged. The temperature is brought at 150°C and the reaction mixture is left under stirring in these conditions for about 10 hours.

At the end of the reaction, 50 mL of water and 50 mL of ethyl acetate are added, the phases are separated and the aqueous phase is extracted with 20 mL of ethyl acetate. Then, the organic phases are combined and washed with water (3 x 50 mL) and brine (1 x 20 mL).

The collected organic phase is filtered on celite and charcoal and then dried with magnesium sulphate. After evaporation of the solvent, 3.40 g of (S)-6-(benzyloxy)-2-(bromomethyl)-2,8-dimethylchromane is obtained (98.0%).

### EXAMPLE 5 Synthesis of (S)-2,8-dimethyl-2-((tosyloxy)methyl)chroman-6-yl 4-methylbenzenesulfonate.

In a reaction flask 0.5 g of 8-methylcroman-2-methanol (MCM, 1 eq), 5.0 mL of dichloromethane (10 vol), 0.73 g of triethylamine (3 eq) and 0,05 4-dimethylaminopyridine (DMAP, 0.2 eq) are charged. 1.09 g of tosyl chloride (2.4 eq) is added and the reaction mixture is left under stirring at room temperature overnight.

At the end of the reaction, 5 mL of dichloromethane and 10 mL of 1 N solution of hydrochloric acid are added. The phases are separated and the organic phase is washed with water (2 x 10 mL) to reach a pH value of 6. The collected organic phase is dried with magnesium sulphate. After evaporation of the solvent, 1.20 g of (S)-2,8-dimethyl-2-((tosyloxy)methyl)chroman-6-yl 4-methylbenzenesulfonate is obtained (97%).

¹H-NMR (CDCl₃, 400 MHz): δ 7.78-7.76 (d, 2H), δ 7.73-7.70 (d, 2H), δ 7.35-7.31 (m, 4H), 6.55-6.52 (d, 2H), δ 4.00-3.90 (dd, 2H), δ 2.63-2.59 (m, 2H), δ 2.45 (s, 6H), δ 1.98 (s, 3H), δ 1.89-1.87 (m, 1H), δ 1.73-1.70 (m, 1H), δ 1.27 (s, 3H).

¹³C-NMR (CDCl₃, 400 MHz): δ 149.63, δ 145.19, δ 145.10, δ 141.98, δ 132.66, δ 132.58, δ 129.69, δ 129,68, δ 128.47, δ 127.87, δ 127.63, δ 122.27, δ 120.71, δ 120.11, δ 74.60, δ 73.35, δ 27.59, δ 22.21, δ 21.70, δ 21.65, δ 21.55, δ 15.91.

### EXAMPLE 6. Synthesis of (S)-2-(iodomethyl)-2,8-dimethylchroman-6-yl 4-methylbenzenesulfonate

In a reaction flask 24.8 g of (S)-2,8-dimethyl-2-((tosyloxy)methyl)chroman-6-yl 4-methylbenzenesulfonate (1 eq), 250 mL of dimethylformamide (10 vol), 39.8 g of potassium iodide (5 eq) are charged. The temperature is brought at 150 °C and the reaction mixture is left under stirring in these conditions for about 10 hours.

At the end of the reaction, 250 mL of water and 250 mL of ethyl acetate are added, the phases are separated and the aqueous phase is extracted with 200 mL of ethyl acetate. Then, the organic phases are combined and washed with water (3 x 250 mL) and brine.

The collected organic phase is filtered on celite and charcoal and then dried with magnesium sulphate. After evaporation of the solvent, 20.9 g of (S)-2-(iodomethyl)-2,8-dimethylchroman-6-yl 4-methylbenzenesulfonate is obtained (92%).

¹H-NMR (CDCl₃, 400 MHz): δ 7.74-7.72 (d, 2H), δ 7.34-7.32 (d, 2H), δ 6.59-6.56 (d, 2H), δ 3.32 (m, 2H), δ 2.66 (m, 2H), δ 2.46 (s, 3H), δ 2.10 (s, 3H), δ 2.06 (m, 1H), δ 1.89 (m, 1H), δ1.45 (s, 3H).

¹³C-NMR (CDCl₃, 400 MHz): δ 149.94, δ 145.11, δ 141.98, δ 132.77, δ 129.65, δ 128.50, δ 127.85, δ 122.24, δ120.76, δ 120.16, δ 75.22, δ 29.79, δ 25.32, δ 21.92, δ 21.72, δ 16.09, δ 14.76

### EXAMPLE 7. Synthesis of (2E,6E)-1-bromo-3,7,11-trimethyldodeca-2,6,10-triene.

In a reaction flask 5.0 g of trans,trans-farnesol (1 eq), 25 mL of tetrahydrofuran (5 vol) are charged. The reaction mixture is cooled to 0 °C and 2.8 g of phosphorus tribromide (0.46 eq) are added dropwise for about 40 minutes. The reaction mixture is left under stirring in these conditions for about 3 hours.

Once the reaction is over, 12.5 mL of water are added dropwise, maintaining the temperature at 0 °C. After bringing the reaction to room temperature, 12.5 mL of methyl-tetrahydrofuran are added and the phases are separated. The aqueous phase is extracted with 10 mL of methyl-tetrahydrofuran and the combined organic phases are washed with a saturated solution of sodium bicarbonate (3 x 250 mL) and brine (1 x 5 mL).

The collected organic phase is dried with magnesium sulphate and the solvent is evaporated giving 6.4 g of (2E,6E)-1-bromo-3,7,11-trimethyldodeca-2,6,10-triene (99.8%).

¹H-NMR (CDCl₃, 400 MHz): δ 5.55 (t, 1H), δ 5.12-5.09 (m, 2H), δ 4.05-4.03 (d, 2H), δ 2.13-1.99 (m, 8H), δ 1.75 (s, 3H), δ 1.70 (s, 3H), δ 1.62 (s, 6H).

¹³C-NMR (CDCl₃, 400 MHz): δ 143.55, δ 135.59, δ 131.28, δ 124.34, δ 123.40, δ 120.61, δ 39.69, δ 39.53, δ 29.60, δ 26.71, δ 26.11, δ 25.71, δ 17.71, δ 16.05, δ 15.97

### EXAMPLE 8. Synthesis of (R)-2,8-dimethyl-2-((3E,7E)-4,8,12-trimethyltrideca-3,7,11-trien-1-yl)chroman-6-yl 4-methylbenzenesulfonate

In a reaction flask 32.0 g of magnesium turnings (6 eq), 200 mL of methyl-tetrahydrofuran (2 vol) are charged under nitrogen atmosphere. The reaction mixture is slowly warmed up to 70 °C and a solution of 105.0 g of (S)-6-(benzyloxy)-2-(iodomethyl)-2,8-dimethylchromane (1 eq) and (2E,6E)-1-bromo-3,7,11-trimethyldodeca-2,6,10-triene (76.0 g 1.2 eq) in 300 ml of methyl-tetrahydrofuran (3 vol) are added dropwise for about 1.30 hours. The reaction mixture is left under stirring at reflux in these conditions for about 1 hour.

Once the reaction is over, the reaction is brought to room temperature and the solid magnesium turnings is removed. The solvent is evaporated and the crude (R)-2,8-dimethyl-2-((3E,7E)-4,8,12-trimethyltrideca-3,7,11-trien-1-yl)chroman-6-yl 4-methylbenzenesulfonate is extracted with exhane (2 x 250 mL). The organic phases are combined and after the evaporation of the solvent the crude product obtained is taken as such for the next step.

¹H-NMR (CDCl₃, 400 MHz): δ 7.75-7.73 (d, 2H), δ 7.33-7.31 (d, 2H), δ 6.56 (s, 2H), δ 5.13 (m, 3H), δ 2.67 (m, 2H), δ 2.46 (s, 3H), δ 2.09 (m, 9H), δ 2.01 (m, 4H), δ1.76 (m, 2H) δ 1.70 (m, 3H), δ 1.62 (m, 9H), δ 1.28 (m, 5H).

¹³C-NMR (CDCl₃, 400 MHz): δ 150.67, δ 144.96, δ 141.43, δ 135.30, δ 134.97, δ 132.89, δ 131.20, δ 129.57, δ 128.53, δ 127.43, δ 124.37, δ 124.16, δ 124.06, δ 121.89, δ 121.09, δ 120.18, δ 76.07, δ 39.81, δ 39.73, δ 39.69, δ 30.88, δ 26.77, δ 26.58, δ 25.71, δ 23.99, δ 22.31, δ 22.12, δ 21.67, δ 17.69, δ 16.04, δ 16.02, δ 15.88.

### EXAMPLE 9. Coupling reaction for the (R)-2,8-dimethyl-2-((3E,7E)-4,8,12-trimethyltrideca-3,7,11-trien-1-yl)chroman-6-yl 4-methylbenzenesulfonate (comparative)

The coupling reaction is carried out following the experimental conditions disclosed in the prior art WO 2005/035490 (Example 1 - step 2). There is no evidence about the formation of the desired product.

### EXAMPLE 10. Synthesis of δ-tocotrienol

In a reaction flask 15.5 g of crude (R)-2,8-dimethyl-2-((3E,7E)-4,8,12-trimethyltrideca-3,7,11-trien-1-yl)chroman-6-yl 4-methylbenzenesulfonate (1 eq), 140 mL of tetrahydrofuran (9 vol), 15 mL of water (1 vol), 19,5 g of potassium carbonate (5 eq) are charged under nitrogen atmosphere. The reaction mixture is left under stirring at reflux in these conditions overnight

Once the reaction is over, the reaction is brought to room temperature and the solvent is removed by evaporation. The crude product is dissolved in methyl-tetrahydrofuran (10 vol) and water (10 vol), the phases are separated and the organic one is washed with brine and dried with magnesium sulfate. The solvent is evaporated and 10.8 g of δ-tocotrienol are obtained (97%)

¹H-NMR (CDCl₃, 400 MHz): δ 6.52 - 6.51 (d, 1H), δ 6.43 - 6.42 (d, 1H), δ 5.19 - 5.15 (m, 3H), δ 5.05 (s, 1H), δ 2.74 - 2.71 (t, 2H), δ 2.17 - 2.10 (m, 9H), δ 2.02 (m, 4H), δ 1.85 - 1.82 (m, 2H), δ 1.80 (m, 4H) δ 1.78 - 1.73 (m, 10H), δ 1.31 (m, 3H).

¹³C-NMR (CDCl₃, 400 MHz): δ 148.17, δ 147.75, δ 145.97, δ 135.15, δ 134.99, δ 131.28, δ 127.35, δ 124.46, δ 124.34, δ 124.24, δ 121.28, δ 115.78, δ112.73, δ 75.37, δ 39.75, δ 39.73, δ 31.42, δ 26.79, δ 26.63, δ 25.73, δ 24.04, δ 22.51, δ 22.21, δ 17.72, δ 16.09, δ 16.04, δ 15.91.

## Claims

1. Process for the preparation of δ-tocotrienol of formula (I) 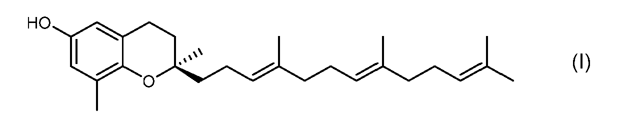 comprising the following steps:
c) reacting a compound of formula (V) 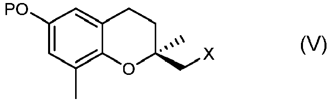
with a compound of formula (VII) 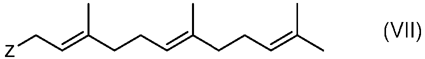
wherein P represents a protecting group selected from benzoyl, acetyl, trimethylsilyl, tert-butyldimethylsilyl, tert-butyldiphenylsilyl, p-toluenesulfonate, methansulfonate, benzensulfonate, p-toluenesulfonate ester, benzensulfonate ester, methanesulfonate ester, benzyl ether, methyl ether, 2-tetrahydropyranyl ether, 2-tetrahydrofuranyl ether, methoxymethyl ether,
X is a halogen atom selected from bromine, iodine, chlorine,
Z represents a halogen atom selected from bromine, iodine, chlorine in the presence of magnesium metal, a magnesium-activating agent and an optional additive,
to provide the compound of formula (VIII) 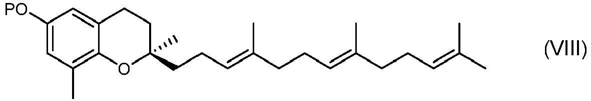 and
d) deprotecting the obtained compound of formula (VIII) to give the desired δ-tocotrienol of formula (I).

2. Process according to claim 1, in which magnesium metal is added in an amount comprised between 1.0 and 10.0 molar equivalents, preferably between 5.0 and 7.0 molar equivalents, with respect to the molar amount of the compound of formula (V).

3. Process according to any one of the preceding claims, in which said magnesium activating agent is selected from iodine, dichloroethane, dibromoethane, trimethylsilyl chloride.

4. Process according to claim 3, in which said magnesium activating agent is added to the reaction mixture in an amount comprised between 0.01 and 1.0 molar equivalents, with respect to the molar amount of the compound of formula (V).

5. Process according to any one of the preceding claims, in which said optional additive is a lithium, zinc, cobalt, nickel, copper, palladium, or iron salt selected from lithium chloride, lithium bromide, lithium iodide, zinc chloride, zinc bromide, zinc iodide, cobalt chloride, cobalt dichloride, nickel chloride, copper(I) chloride, copper(II) chloride, copper(I) bromide, copper(II) dibromide, copper iodide, copper triflate, palladium chloride, iron(III) chloride, preferably lithium chloride, and/or an organic chelating compound selected from tetramethylethylenediamine (TMEDA), 1,2-Dimethylethylenediamine (DMEDA), ethanolamine (ETA), triphenylphosphine (PPh₃), 1,3-bis(diphenylphosphino)propane (DPPP), 1,3-butadiene, isoprene.

6. Process according to any one of the preceding claims, in which the temperature of step c) is comprised between 20°C and 100°C, preferably of about 80°C.

7. Process according to any one of the preceding claims, in which X is iodine and Z is bromine.

8. Process according to any one of the preceding claims, in which the compound of formula (V) is prepared by the following steps:
a) selectively protecting the aromatic hydroxyl group of 8-methylcroman-2-methanol of formula (II) in single enantiomer form 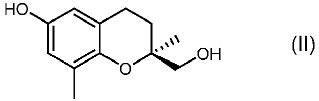 in the presence of a protecting agent, to give the intermediate of formula (IV) 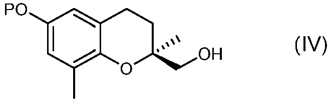 wherein P has the above reported meanings,
b) converting the compound of formula (IV) into the corresponding halide derivative of formula (V) 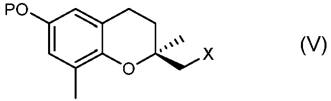 wherein X is a halogen atom selected from bromine, iodine, chlorine.

9. Process according to any one of claims 1-7, in which the compound of formula (V) is prepared by the following steps:
a) selectively protecting the aromatic hydroxyl group of 8-methylcroman-2-methanol of formula (II) in single enantiomer form 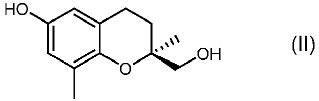 in the presence of a protecting agent, to give the intermediate of formula (IV) 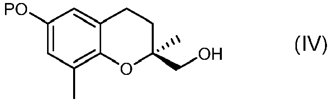 wherein P has the above reported meanings,
b1) converting the compound of formula (IV) Into the corresponding compound of formula (VI) 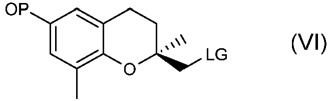
Wherein P has the above-mentioned meanings
and LG represents a sulfonate leaving group selected from tosyl, mesyl, nosyl, triflate, nonaflate, preferably tosyl;
b2) halogenating the compound of formula (VI) thus obtained to provide the desired compound of formula (V)

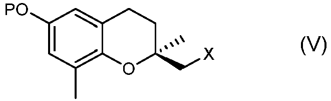

10. Process according to any one of the preceding claims, in which said compound of formula (VII) 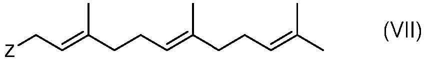 wherein Z represents a halogen atom selected from chlorine, bromine, iodine, is obtained by halogenating the compound of formula (IX) 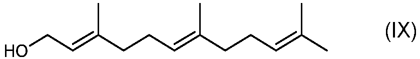 in the presence of a halogenating agent.

## Patentansprüche

1. Verfahren zum Herstellen von δ-Tocotrienol der Formel (I) 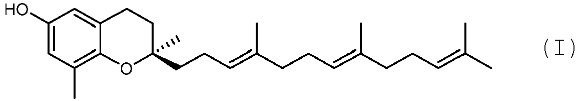 das die folgenden Schritte umfasst:
c) Umsetzen einer Verbindung der Formel (V) 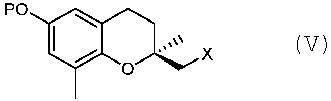
mit einer Verbindung der Formel (VII) 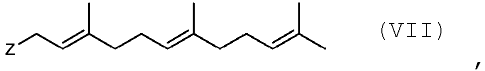
wobei P eine Schutzgruppe darstellt, die aus Benzoyl, Acetyl, Trimethylsilyl, tert-Butyldimethylsilyl, tert-Butyldiphenylsilyl, p-Toluolsulfonat, Methansulfonat, Benzolsulfonat, p-Toluolsulfonatester, Benzolsulfonatester, Methansulfonatester, Benzylether, Methylether, 2-Tetrahydropyranylether, 2-Tetrahydrofuranylether, Methoxymethylether ausgewählt ist,
X ein Halogenatom ist, das aus Brom, Iod, Chlor ausgewählt ist, Z ein Halogenatom darstellt, das aus Brom, Iod, Chlor ausgewählt ist,
in der Gegenwart von Magnesiummetall, eines magnesiumaktivierenden Mittels und eines optionalen Zusatzstoffs,
um die Verbindung der Formel (VIII) 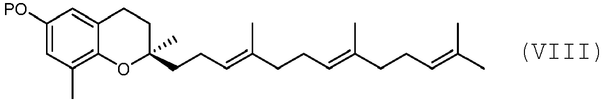 bereitzustellen, und
d) Entschützen der erhaltenen Verbindung der Formel (VIII), um das gewünschte δ-Tocotrienol der Formel (I) zu erhalten.

2. Verfahren nach Anspruch 1, wobei Magnesiummetall in einer Menge zwischen 1,0 und 10,0 Moläquivalenten, vorzugsweise zwischen 5,0 und 7,0 Moläquivalenten, in Bezug auf die Molmenge der Verbindung der Formel (V) hinzugefügt wird.

3. Verfahren nach einem der vorhergehenden Ansprüche, wobei das magnesiumaktivierende Mittel aus Iod, Dichlorethan, Dibromethan, Trimethylsilylchlorid ausgewählt ist.

4. Verfahren nach Anspruch 3, wobei das magnesiumaktivierende Mittel in einer Menge zwischen 0,01 und 1,0 Moläquivalenten in Bezug auf die Molmenge der Verbindung der Formel (V) zu dem Reaktionsgemisch hinzugefügt wird.

5. Verfahren nach einem der vorstehenden Ansprüche, wobei der optionale Zusatzstoff ein Lithium-, Zink-, Kobalt-, Nickel-, Kupfer-, Palladium- oder Eisensalz ist, das aus Lithiumchlorid, Lithiumbromid, Lithiumiodid, Zinkchlorid, Zinkbromid, Zinkiodid, Kobaltchlorid, Kobaltdichlorid, Nickelchlorid, Kupfer(I)chlorid, Kupfer(II)chlorid, Kupfer(I)bromid, Kupfer(II)dibromid, Kupferiodid, Kupfertriflat, Palladiumchlorid, Eisen(III)chlorid, vorzugsweise Lithiumchlorid, ausgewählt ist, und/oder eine organische chelatbildende Verbindung ist, die aus Tetramethylethylendiamin (TMEDA), 1,2-Dimethylethylendiamin (DMEDA), Ethanolamin (ETA), Triphenylphosphin (PPh₃), 1,3-Bis(diphenylphosphino)propan (DPPP), 1,3-Butadien, Isopren ausgewählt ist.

6. Verfahren nach einem der vorstehenden Ansprüche, wobei die Temperatur von Schritt c) zwischen 20 °C und 100 °C liegt, vorzugsweise ungefähr 80 °C beträgt.

7. Verfahren nach einem der vorhergehenden Ansprüche, wobei X Iod ist und Z Brom ist.

8. Verfahren nach einem der vorhergehenden Ansprüche, wobei die Verbindung der Formel (V) mit den folgenden Schritten hergestellt wird:
a) selektives Schützen der aromatischen Hydroxylgruppe von 8-Methylcroman-2-methanol der Formel (II) in Einzelenantiomerform 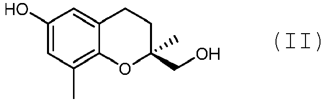 in der Gegenwart eines Schutzmittels, um das Zwischenprodukt der Formel (IV) 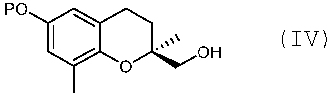 zu erhalten, wobei P die oben angeführten Bedeutungen hat,
b) Umwandeln der Verbindung der Formel (IV) in das entsprechende Halogenidderivat der Formel (V) 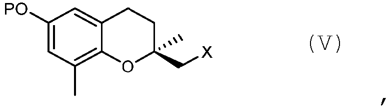 wobei X ein Halogenatom ist, das aus Brom, Iod, Chlor ausgewählt ist.

9. Verfahren nach einem der Ansprüche 1 bis 7, wobei die Verbindung der Formel (V) mit den folgenden Schritten hergestellt wird:
a) selektives Schützen der aromatischen Hydroxylgruppe von 8-Methylcroman-2-methanol der Formel (II) in Einzelenantiomerform 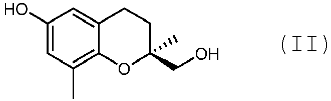 in der Gegenwart eines Schutzmittels, um das Zwischenprodukt der Formel (IV) 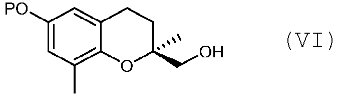 zu erhalten, wobei P die oben angeführten Bedeutungen hat,
b1) Umwandeln der Verbindung der Formel (IV) in die entsprechende Verbindung der Formel (VI) 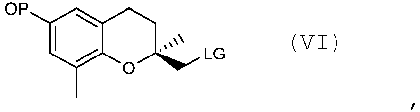 wobei P die oben angeführten Bedeutungen hat und LG eine Sulfonatabgangsgruppe darstellt, die aus Tosyl, Mesyl, Nosyl, Triflat, Nonaflat, vorzugsweise Tosyl, ausgewählt ist;
b2) Halogenieren der auf diese Weise erhaltenen Verbindung der Formel (VI), um die gewünschte Verbindung der Formel (V) 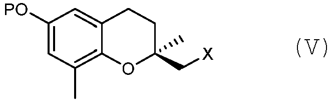 bereitzustellen.

10. Verfahren nach einem der vorhergehenden Ansprüche, wobei die Verbindung der Formel (VII) 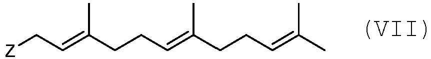 wobei Z ein Halogenatom darstellt, das aus Chlor, Brom, Iod ausgewählt ist, durch Halogenieren der Verbindung der Formel (IX) 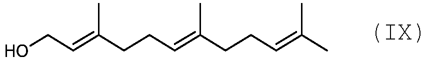 in Gegenwart eines Halogenierungsmittels erhalten wird.

## Revendications

1. Procédé de préparation de δ-tocotriénol de formule (I) 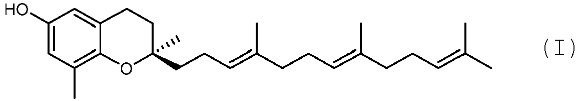 comprenant les étapes suivantes :
c) la réaction d'un composé de formule (V) 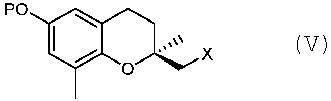
avec un composé de formule (VII) 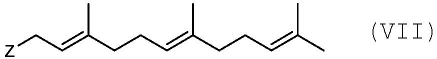
dans lequel P représente un groupe protecteur choisi parmi le benzoyle, l'acétyle, le triméthylsilyle, le tert-butyldiméthylsilyle, le tert-butyldiphénylsilyle, le p-toluènesulfonate, le méthanesulfonate, le benzènesulfonate, l'ester de p-toluènesulfonate, l'ester de benzènesulfonate, l'ester de méthanesulfonate, l'éther benzylique, l'éther méthylique, l'éther 2-tétrahydropyranylique, l'éther 2-tétrahydrofuranylique, l'éther méthoxyméthylique,
X est un atome d'halogène choisi parmi le brome, l'iode, le chlore,
Z représente un atome d'halogène choisi parmi le brome, l'iode, le chlore
en présence de magnésium métallique, d'un agent activateur de magnésium et d'un additif éventuel,
pour fournir le composé de formule (VIII) 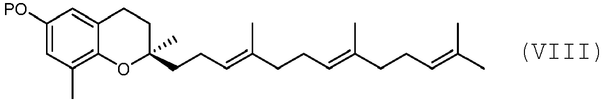 et
d) la déprotection du composé obtenu de formule (VIII) pour donner le δ-tocotriénol souhaité de formule (I).

2. Procédé selon la revendication 1, dans lequel le magnésium métallique est ajouté en une quantité comprise entre 1,0 et 10,0 équivalents molaires, de préférence entre 5,0 et 7,0 équivalents molaires, par rapport à la quantité molaire du composé de formule (V).

3. Procédé selon l'une quelconque des revendications précédentes, dans lequel ledit agent activateur de magnésium est choisi parmi l'iode, le dichloroéthane, le dibromoéthane, le chlorure de triméthylsilyle.

4. Procédé selon la revendication 3, dans lequel ledit agent activateur de magnésium est ajouté au mélange réactionnel en une quantité comprise entre 0,01 et 1,0 équivalent molaire, par rapport à la quantité molaire du composé de formule (V).

5. Procédé selon l'une quelconque des revendications précédentes, dans lequel ledit additif éventuel est un sel de lithium, de zinc, de cobalt, de nickel, de cuivre, de palladium ou de fer choisi parmi le chlorure de lithium, le bromure de lithium, l'iodure de lithium, le chlorure de zinc, le bromure de zinc, l'iodure de zinc, le chlorure de cobalt, le dichlorure de cobalt, le chlorure de nickel, le chlorure de cuivre(I), le chlorure de cuivre(II), le bromure de cuivre(I), le dibromure de cuivre(II), l'iodure de cuivre, le triflate de cuivre, le chlorure de palladium, le chlorure de fer(III), de préférence le chlorure de lithium, et/ou un composé chélatant organique choisi parmi la tétraméthyléthylènediamine (TMEDA), la 1,2-diméthyléthylènediamine (DMEDA), l'éthanolamine (ETA), la triphénylphosphine (PPh₃), le 1,3-bis(diphénylphosphino)propane (DPPP), le 1,3-butadiène, l'isoprène.

6. Procédé selon l'une quelconque des revendications précédentes, dans lequel la température de l'étape c) est comprise entre 20°C et 100°C, de préférence d'environ 80°C.

7. Procédé selon l'une quelconque des revendications précédentes, dans lequel X est l'iode et Z est le brome.

8. Procédé selon l'une quelconque des revendications précédentes, dans lequel le composé de formule (V) est préparé par les étapes suivantes :
a) la protection sélective du groupe hydroxyle aromatique de 8-méthylcroman-2-méthanol de formule (II) sous forme d'énantiomère unique 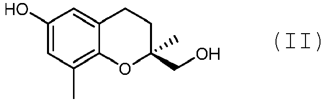 en présence d'un agent protecteur, pour donner l'intermédiaire de formule (IV) 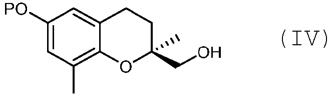 dans lequel P a les significations indiquées ci-dessus,
b) la conversion du composé de formule (IV) en le dérivé halogénure correspondant de formule (V) 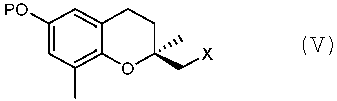 dans lequel X est un atome d'halogène choisi parmi le brome, l'iode, le chlore.

9. Procédé selon l'une quelconque des revendications 1 à 7, dans lequel le composé de formule (V) est préparé par les étapes suivantes :
a) la protection sélective du groupe hydroxyle aromatique de 8-méthylcroman-2-méthanol de formule (II) sous forme d'énantiomère unique 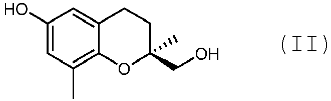 en présence d'un agent protecteur, pour donner l'intermédiaire de formule (IV) 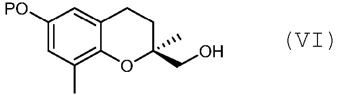 dans lequel P a les significations indiquées ci-dessus,
b1) la conversion du composé de formule (IV) en le composé correspondant de formule (VI) 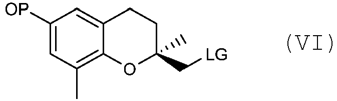 dans lequel P a les significations mentionnées ci-dessus et LG représente un groupe partant sulfonate choisi parmi tosyle, mésyle, nosyle, triflate, nonaflate, de préférence tosyle ;
b2) l'halogénation du composé de formule (VI) ainsi obtenu pour obtenir le composé de formule (V) souhaité

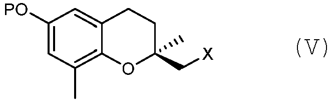

10. Procédé selon l'une quelconque des revendications précédentes, dans lequel ledit composé de formule (VII) 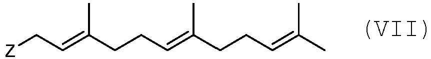 dans lequel Z représente un atome d'halogène choisi parmi le chlore, le brome, l'iode, est obtenu par halogénation du composé de formule (IX) 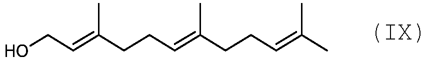 en présence d'un agent halogénant.
